# EUROPEAN PATENT APPLICATION

(11) **EP 4 628 573 A1**
(43) Date of publication of application: **08.10.2025**
(21) Application number: 23907033.7
(22) Date of filing: 19.12.2023
(51) Int. Cl.: C12N 5/0775, A61K 33/00, A61K 33/18, A61K 35/28, A61K 51/00, A61P 35/00, A61P 35/04, C12N 5/10

(54) **DENTAL PULP-DERIVED STEM CELLS EXPRESSING SODIUM IODIDE SYMPORTER**

(30) Priority: 20.12.2022 JP 2022203175
(71) Applicant: The University of Tokyo, Bunkyo-ku, Tokyo 113-8654 (JP); JCR Pharmaceuticals Co., Ltd., Ashiya-shi, Hyogo 659-0021 (JP)
(72) Inventor: NOMURA Sachiyo, Tokyo 113-8654 (JP); NUMATA Ryohei, Kobe-shi, Hyogo 651-2241 (JP); ISHIKAWA Hiroki, Kobe-shi, Hyogo 651-2241 (JP); YOKOYAMA Wataru, Kobe-shi, Hyogo 651-2241 (JP); IMAGAWA Kiwamu, Kobe-shi, Hyogo 651-2241 (JP)
(74) Representative: Isarpatent
(86) International application number: PCT/JP2023/045482
(87) International publication number: WO 2024/135673

(57) **Abstract**

It is an object of the present invention to provide cells, which accumulate in cancer tissues, are guaranteed to be safe for humans, and can be stably prepared. Moreover, it is another object of the present invention to provide a method for diagnosing a cancer and a method for treating a cancer, using the above-described cells expressing NIS, and to provide a composition for use in the diagnostic method and the treatment method. More specifically, the present invention relates to: dental pulp-derived pluripotent stem cells, which express a sodium iodide symporter (NIS); a composition for use in the treatment or diagnosis of a cancer, which is characterized in that the aforementioned cells are combined with a radionuclide; and a method for treating a cancer and a method for diagnosing a cancer, using the aforementioned composition.

## Description

### Technical Field

The present invention relates to dental pulp stem cells that express a sodium iodide symporter (NIS), and a method for diagnosing and treating a cancer using the dental pulp stem cells.

### Background Art

Currently, cancer therapy is carried out by a combination of various methods, including a surgical treatment such as surgery, a chemotherapy using anticancer drugs, a radiation therapy and immune checkpoint inhibitors, and the survival rate of cancer patients is improving. However, such existing treatment methods often do not provide satisfactory therapeutic effects on refractory cancers such as scirrhous stomach cancer, pancreatic cancer and triple-negative breast cancer, as well as on peritoneal dissemination, etc. that occurs as a result of metastasis from digestive cancers such as stomach cancer, pancreatic cancer and colon cancer, and also, ovarian and ureteral cancer. In particular, peritoneal dissemination is difficult to be removed by surgical operation, and a treatment with conventional chemotherapeutic agents alone has poor therapeutic effects, resulting in an extremely poor prognosis.

It is being attempted to develop a novel cancer treatment method focused on the tumor microenvironment (TME). What is called "cancer" is composed of tumor tissues consisting of tumor cells, and cancer stroma consisting of stromal fibroblasts, cells that form blood vessels and lymphatic vessels, infiltrative inflammatory cells, extracellular matrixes such as collagen, and physiologically active substances. Cancer stroma grows by high accumulation of stromal cells and proliferation thereof, and during this growth process, mesenchymal stem cells (MSCs) are incorporated in the cancer stroma. MSCs are characterized by high proliferation and have an ability to differentiate into cells that constitute connective tissues such as bone, fat, cartilage and muscle, and such MSCs play an important role in the maintenance and regeneration of various tissues.

In recent years, by utilizing the ability of bone marrow-derived MSCs to accumulate in the cancer stroma, a cancer treatment method, etc., which comprise delivering a cancer therapeutic gene, etc. to the tumor microenvironment, have been proposed (see, for example, Non Patent Literature 1, etc.). In particular, a radiation therapy, in which bone marrow-derived MSCs are combined with a radionuclide, has attracted attention. It has been reported that bone marrow-derived MSCs expressing a sodium iodide symporter (NIS) can also be used to deliver a radionuclide to the TME and to kill cancer cells (Non Patent Literature 2, etc.). NIS is a transmembrane glycoprotein having 13 transmembrane domains and can transport radionuclides such as ¹³¹I, ¹²³I, ¹²⁵I, ¹²⁴I, ^{99m}Tc, ¹⁸⁸Re and ²¹¹At. For example, Non Patent Literature 2 reports that administration of NIS-expressing MSCs and ¹³¹I to liver cancer mouse models resulted in tumor degeneration.

As described above, a treatment method of using bone marrow-derived MSCs expressing NIS as carriers to deliver a radionuclide to a cancer lesion is a method that is greatly expected to have excellent therapeutic effects. However, there are problems to be solved in stable preparation of bone marrow-derived MSCs, due to the reasons that the number of stem cells in the bone marrow decreases with aging, and that bone marrow puncture for obtaining a bone marrow fluid causes a heavy burden on the body.

### Citation List

### Patent Literature

Patent Literature 1: WO2020/027163
Patent Literature 2: WO2002/007679
Patent Literature 3: JP Patent Publication (Kokai) No. 2010-252778A
Patent Literature 4: JP Patent Publication (Kohyo) No. 2008-507962 A
Patent Literature 5: WO2009/072527
Patent Literature 6: JP Patent Publication (Kokai) No. 2004-201612 A

### Non Patent Literature

Non Patent Literature 1: Loebinger et al., Cancer Res. 69: 4134-4142, 2009.
Non Patent Literature 2: Knoop et al., Molecular Therapy 19: 1704-1713, 2011.
Non Patent Literature 3: Gronthos et al., Proc Natl Acad Sci USA, 97: 13625-13630, 2000.
Non Patent Literature 4: Spitzweg and Morrist, Clin Endocrinol 57: 559-574, 2002.
Non Patent Literature 5: Yamamoto et al., Cancer Res. 109: 1480-1492, 2018.

### Summary of Invention

### Technical Problem

In view of the above circumstances, it is an object of the present invention to provide cells that accumulate in cancer tissues and that can be prepared more stably than bone marrow-derived MSCs.

Moreover, it is another object of the present invention to provide a method for diagnosing a cancer and a method for treating a cancer, using the above-described cells expressing NIS, and to provide a composition for use in the diagnostic method and the treatment method.

### Solution to Problem

The present inventors have conducted intensive studies regarding cells that can be used as carriers for delivering a radionuclide to cancer tissues, instead of bone marrow-derived MSCs, and that contribute to solving the above-described problems, and as a result, they have focused on dental pulp stem cell (DPC).

It has already been known that pluripotent stem cells can be obtained from dental pulp (see, for example, Patent Literature 1 to Patent Literature 6 and Non Patent Literature 3, etc.), and it has been reported that DPCs have a higher cell proliferation ability than bone marrow-derived MSCs (Non Patent Literature 3). In addition, it is considered that if the donor's consent is obtained, dental pulp stem cells contained in baby teeth and permanent teeth can be easily collected non-invasively, and there are few ethical and safety issues.

First, the present inventors have prepared DPCs that stably express human NIS (hereinafter referred to as "hNIS-DPCs"). When the amount of iodine uptake by hNIS-DPCs was compared with that by hNIS-MSCs, it was revealed that the amount of iodine uptake by hNIS-DPCs was approximately 2 to 4 times higher than that by hNIS-MSCs. Therefore, it was suggested that the amount of a radionuclide uptake by hNIS-DPCs is also higher than that by hNIS-MSCs, and that hNIS-DPCs have extremely high effects of cancer treatment.

Moreover, the present inventors have administered hNIS-DPCs into the abdominal cavity or vein of peritoneal dissemination mouse models, and as a result, they have found that the cells accumulated in the vicinity of the peritoneal dissemination of stomach cancer. Furthermore, the present inventors have found that when the cells were administered to peritoneal dissemination mouse models and then, a radionuclide such as ¹³¹I or ²¹¹At was administered thereto, the cells that accumulated in the vicinity of the peritoneal dissemination took up such a radionuclide and then delivered the radionuclide to the peritoneal dissemination tissues, so that the radionuclide damaged and killed the tumor cells. From the aforementioned results, it was demonstrated that cancer such as peritoneal dissemination can be discovered and treated by using DPCs expressing NIS (also referred to as "NIS-DPCs").

Specifically, the present invention includes the following (1) to (12).
(1) Dental pulp stem cells, which express a sodium iodide symporter (NIS).
(2) The cells according to the above (1), which are derived from a human dental pulp.
(3) The cells according to the above (1), wherein the NIS is introduced from the outside.
(4) A composition comprising the cells according to any one of the above (1) to (3), wherein the composition is characterized in that it is used in combination with a radionuclide.
(5) The composition according to the above (4), which is a pharmaceutical composition for use in the treatment of a cancer.
(6) The composition according to the above (5), which is characterized in that the composition is intratumorally, intraperitoneally or intravenously administered, and the radionuclide is intratumorally, intraperitoneally, intravenously or orally administered.
(7) The composition according to the above (5), wherein the radionuclide is any of ¹³¹I, ²¹¹At or ¹⁸⁸Re.
(8) The composition according to the above (5), wherein the cancer is a peritoneal dissemination.
(9) The composition according to the above (4), which is a composition for use in the diagnosis of a cancer.
(10) The composition according to the above (9), which is characterized in that the composition is intratumorally, intraperitoneally or intravenously administered, and the radionuclide is intratumorally, intraperitoneally, intravenously or orally administered.
(11) The composition according to the above (9), wherein the radionuclide is any of ¹³¹I, ¹²³I, ¹²⁵I, ¹²⁴I or ^{99m}Tc.
(12) The composition according to the above (9), wherein the cancer is a peritoneal dissemination.

It is to be noted that the preposition "to" used in the present description indicates a numerical value range including the numerical values located left and right of the preposition.

### Advantageous Effects of Invention

According to the present invention, NIS-DPCs, which can exhibit higher cancer treatment effects than NIS-MSCs, are provided. Thereby, it becomes possible to provide a treatment method having higher effects than conventional cancer treatment methods using a radionuclide.

### Brief Description of Drawings

[Figure 1] Figure 1 shows a transition in the doubling days of hNIS-DPCs after lentivirus infection of DPCs. The vertical axis indicates the doubling days of hNIS-DPCs, and the horizontal axis indicates the accumulated PDL of hNIS-DPCs.
[Figure 2] Figure 2 shows a transition in hNIS-positive percentage during continuous culture of hNIS-DPCs, which have been prepared by infecting DPCs with lentivirus using protamine or polybrene. The vertical axis indicates the hNIS-positive percentage, and the horizontal axis indicates the number of passages of hNIS-DPCs.
[Figure 3] Figure 3 shows the relationship between the MOI (multiplicity of infection) at the time of infection with lentivirus (3 lots) having an hNIS coding sequence, and the hNIS-positive percentage when the cells were harvested after 2 passages. The vertical axis indicates the hNIS-positive percentage, and the horizontal axis indicates the multiplicity of infection (MOI).
[Figure 4] Figure 4 shows the relationship between the NIS-positive percentage of hNIS-DPCs and the amount of iodine uptake. The vertical axis indicates the amount of iodine uptake (pmol) per 1 x 10⁶ cells, and the horizontal axis indicates the NIS-positive percentage.
[Figure 5] Figure 5 shows the results obtained by comparing the amount of iodine uptake by hNIS-DPCs and that by hNIS-mouse MSCs. The vertical axis indicates the amount of iodine uptake (pmol) per 1 x 10⁶ cells.
[Figure 6] Figure 6 shows the results obtained by intraperitoneally administering DPCs (intact) to peritoneal dissemination mouse models, and then immunostaining peritoneal tissues with an anti-human Lamin B1 antibody. The area surrounded by the dashed line shows cancer tissues. The arrow indicates an immunostaining-positive site derived from DPCs. i.p.: intraperitoneal (intraperitoneal administration)
[Figure 7] Figure 7 shows the results obtained by intravenously administering DPCs to peritoneal dissemination mouse models, and then immunostaining peritoneal tissues with anti-human Lamin B1 antibody. The area surrounded by the dashed line shows cancer tissues. The arrow indicates an immunostaining-positive site derived from DPCs. i.v.: intravenous (intravenous administration)
[Figure 8] Figure 8 shows the experimental schedule for evaluating the migration and accumulation into cancer tissues of hNIS-DPCs administered into the abdominal cavity of peritoneal dissemination mouse models.
[Figure 9] Figure 9 shows the results obtained by evaluating by PCR the migration into cancer tissues of hNIS-DPCs administered into the abdominal cavity of peritoneal dissemination mouse models. The band indicated with the arrow is a band (203 bp) specifically amplified by human GAPDH.
[Figure 10] Figure 10 shows the results obtained by intraperitoneally administering hNIS-DPCs to peritoneal dissemination mouse models, and then immunostaining peritoneal tissues with an anti-human Lamin B1 antibody. In the present experiment, frozen cells were administered as were to the mouse models. The area surrounded by the dashed line shows cancer tissues. The arrow indicates an immunostaining-positive site derived from hNIS-DPCs. i.p.: intraperitoneal (intraperitoneal administration)
[Figure 11] Figure 11 shows the results obtained by intraperitoneally administering hNIS-DPCs to peritoneal dissemination mouse models, and then immunostaining peritoneal tissues with an anti-human Lamin B1 antibody. In the present experiment, frozen cells were thawed, were then pre-cultured, and were then administered. The area surrounded by the dashed line shows cancer tissues. The arrow indicates an immunostaining-positive site derived from hNIS-DPCs. i.p.: intraperitoneal (intraperitoneal administration)
[Figure 12] Figure 12 shows the results obtained by intravenously administering hNIS-DPCs to peritoneal dissemination mouse models, and then immunostaining peritoneal tissues with an anti-human Lamin B1 antibody. In the present experiment, frozen cells were thawed, and were then administered without subjecting to a culture step. The area surrounded by the dashed line shows cancer tissues. The arrow indicates an immunostaining-positive site derived from hNIS-DPCs. i.v.: intravenous (intravenous administration)
[Figure 13] Figure 13 shows the experimental schedule for evaluating migration of hNIS-DPCs to cancer tissues and accumulation of ¹²⁵I, when hNIS-DPCs were intraperitoneally administered and ¹²⁵I was orally administered to peritoneal dissemination mouse models.
[Figure 14] Figure 14 shows the results of measuring the γ radiation dose in major organs, when hNIS-DPCs was intraperitoneally administered and ¹²⁵I was orally administered to peritoneal dissemination mouse models. The vertical axis indicates CPM (count per minute).
[Figure 15] Figure 15 shows the experimental schedule for optimizing the dose of ²¹¹At administered.
[Figure 16] Figure 16 shows the results obtained by visually evaluating and scoring the state of peritoneal dissemination in mice administered with hNIS-DPCs and ²¹¹At.
[Figure 17] Figure 17 shows representative images of the state of peritoneal dissemination in mice administered with hNIS-DPCs and ²¹¹At. The images show the state of the right peritoneum. The numbers in the images are the scores of visual evaluation. 4: Large tumor particles present over the entire surface, 3: tumor particles present but not over the entire surface, 2: tumor particles are clearly present but small, 1: slight tumor particles can be confirmed upon close observation, 0: no tumor particles.
[Figure 18] Figure 18 shows the results obtained by evaluating the state of peritoneal dissemination in mice administered with hNIS-DPCs and ²¹¹At, using the expression level of an FGFR4 gene derived from cancer cells as an indicator.
[Figure 19] Figure 19 shows the experimental schedule for confirming the hNIS-DPC dose (number of administrations) dependency of the antitumor effect on peritoneal dissemination in mice.
[Figure 20] Figure 20 shows the results obtained by visually evaluating and scoring the state of peritoneal dissemination in mice administered with ²¹¹At and hNIS-DPCs (1, 2, or 3 times).
[Figure 21] Figure 21 shows the results obtained by evaluating the survival period of peritoneal dissemination mouse models administered with hNIS-DPCs and ²¹¹At, using a Kaplan-Meier curve and a Log-rank test.
[Figure 22] Figure 22 shows the results obtained by evaluating the survival rate of peritoneal dissemination mouse models administered with hNIS-DPCs and ²¹¹At.
[Figure 23] Figure 23 shows the results obtained by visually evaluating and scoring the peritoneal dissemination state of the peritoneal dissemination mouse models used in the evaluations of Figure 21 and Figure 22.

### Description of Embodiments

Hereafter, the embodiments of the present invention will be described.

A first embodiment relates to dental pulp stem cells (DPCs), which express a sodium iodide symporter (NIS) (hereinafter also referred to as "NIS-DPCs").

NIS is an integral membrane protein having 13 transmembrane domains, which functions as an ion pump and simultaneously transports one iodide ion (I⁻) and two sodium ions (Na+) into cells. To date, the utility of NIS as a new tool in disease treatment has been reported (see, for example, Non Patent Literature 4). The NIS-DPCs according to the present embodiment may be prepared by introducing exogenous NIS into DPCs from the outside. The exogenous NIS used in the present embodiment may be selected from any animal species, depending on the intended use thereof. For example, human NIS consisting of the amino acid sequence as set forth in SEQ ID No: 1 is preferable, but the exogenous NIS used herein is not limited to this sequence. Moreover, in addition to naturally occurring NIS, the exogenous NIS used herein may be a variant or homolog thereof (for example, a variant or homolog of NIS consisting of an amino acid sequence having an identity of 90% or more to the amino acid sequence of naturally occurring NIS and having the activity of simultaneously transporting an iodide ion and sodium ions into cells, etc.), and for example, NIS variants disclosed in US8852576B2, etc. may be used.

DPCs are stem cells present in dental pulp tissues, which have been discovered by Gronthos et al. (Non Patent Literature 3). It has already been known that pluripotent stem cells can be obtained from dental pulp, and can be obtained, for example, by the methods disclosed in Patent Literature 1 to Patent Literature 6.

DPCs can be obtained by culturing cells collected from dental pulp, selecting the cells using a DPC surface antigen marker as an indicator, and finally isolating them. The cell population collected from dental pulp comprises a mixture of DPCs and other cells, but since DPCs have a higher proliferation speed than other cells, the abundance ratio of DPCs is increased at completion of the culture. Therefore, by repeatedly culturing the cell population collected from dental pulp, the abundance ratio of DPCs increases, and cells containing almost only DPCs can be obtained.

When DPCs are selected from a cell population derived from dental pulp, the cells are selected, for example, using, as an indicator, the characteristics shown by at least one of the following characteristics (1) to (4), and the selected cells may be finally used as DPCs in the present embodiment, without any particular limitation:
(1) CD73, CD90, CD105 and CD166 are positive, and CD34, CD40, CD45, CD80, CD86 and the MHC class II antigen are negative, but the MHC class II antigen becomes positive when the cells are stimulated with interferon-γ, and prostaglandin E2 and/or vascular endothelial growth factors are expressed, and the expression level of prostaglandin E2 is increased when the cells are stimulated with TNFα;
(2) At least one of CD73, CD90, CD105 and CD166 is positive, and CD34 and CD45 are negative;
(3) At least one of CD47, CD81, and CD147 is positive, and at least one of CD19, CD34 and CD206 is negative; and
(4) At least one of CD47, CD81 and CD147 is positive, and at least one of CD19, CD31, CD33, CD34, CD38, CD45, CD206, CD235a and SSEA-1 is negative.

For details regarding the method of preparing the above-exemplified DPCs, see Patent Literature 1. It is to be noted that, in the present embodiment, the DPCs may be modified depending on the intended use, and the DPCs may be immortalized using, for example, an SV40 large T antigen, etc. In addition, the animal from which the DPCs used in the present embodiment are derived is not particularly limited, and human-derived DPCs are most preferable.

The expression of exogenous NIS in DPCs can be easily carried out based on common technical knowledge in the present technical field. The vector that can be used to introduce exogenous NIS into DPCs is not particularly limited, and examples thereof may include viral vectors such as a retrovirus vector (e.g., a lentivirus vector), an adenovirus vector, an adeno-associated virus vector, a simian virus 40 (SV40) vector, and a herpes virus vector, as well as non-viral plasmid vectors. Examples of the method of gene introduction using a non-viral vector may include the calcium phosphate method, an electroporation method, and a particle gun method. The promoter that can be used to express NIS in DPCs may be either a constitutive promoter or an inducible promoter. Examples of such a promoter may include, but are not limited to, an E2F promoter, a telomerase (hTERT) promoter, a cytomegalovirus-derived promoter (CMV IE promoter), and an EF1-α promoter. Furthermore, an enhancer may be linked to the promoter to make the transcription activity of the promoter more efficient.

The expression of exogenous NIS in DPCs using a lentivirus vector can be easily carried out based on common technical knowledge in the present technical field. Selection of auxiliary reagents to be used for lentivirus infection and their optimal amounts used can be determined, as appropriate, by preliminary experiments, etc. For example, as infection reagents used in preparation of the NIS-DPCs according to the present embodiment, polybrene and protamine can be used, although there is no particular limitation thereto, and protamine can be particularly preferably used.

A second embodiment relates to a composition comprising DPCs that express NIS (NIS-DPCs), wherein the composition is characterized in that it is used in combination with a radionuclide (hereinafter also referred to as "the composition according to the present embodiment").

The composition according to the present embodiment can be used, for example, for cancer treatment or cancer diagnosis, and the present composition is characterized in that it is combined with a radionuclide and in that the composition and the radionuclide are administered, separately. As described above, since NIS-DPCs accumulate in cancer stroma and also incorporate a radionuclide into the cells, the NIS-DPCs enable the delivery of the radionuclide to cancer tissues (cancer lesion). Thus, by identifying the location of the radionuclide, the site of the cancer lesion can be identified. In addition, by using a nuclide having cytotoxicity, radiation from NIS-DPCs can damage and even kill tumor cells in the vicinity. Therefore, the composition according to the present embodiment can be used for cancer diagnosis or cancer treatment.

The radionuclide used in combination with the composition according to the present embodiment may be any nuclide that can be taken up by the NIS-DPCs, and is not particularly limited. Examples of the radionuclide used herein may include ¹³¹I, ¹²³I, ¹²⁵I, ¹²⁴I, ^{99m}Tc, ¹⁸⁸Re and ²¹¹At. When the composition according to the present embodiment is used for cancer treatment, the radionuclide is preferably a nuclide that emits an α ray or a β ray, which has high cell killing ability, and for example, ¹³¹I, ¹⁸⁸Re or ²¹¹At is preferable. In addition, when the composition according to the present embodiment is used for cancer diagnosis, the radionuclide is preferably a nuclide that can be detected by, for example, SPECT (Single Photon Emission Computed Tomography) examination or PET (Positron Emission Tomography) examination, etc., and for example, a nuclide that emits a γ ray, such as, for example, ¹²³I, ¹²⁵I, ¹²⁴I or ^{99m}Tc is preferable.

When the composition according to the present embodiment is used for the treatment of cancer, the type of the cancer (malignant tumor/neoplasm) as a treatment target is not particularly limited. Examples of the cancer (malignant tumor/neoplasm) as a treatment target may include hepatocellular carcinoma, cholangiocarcinoma, renal cell carcinoma, squamous cell carcinoma, basal cell cancer, transitional cell carcinoma, glandular cancer, malignant gastrinoma, malignant melanoma, fibrosarcoma, myxosarcoma, liposarcoma, leiomyosarcoma, rhabdomyosarcoma, malignant teratoma, angiosarcoma, Kaposi's sarcoma, osteosarcoma, chondrosarcoma, lymphangiosarcoma, malignant meningioma, non-Hodgkin's lymphoma, Hodgkin's lymphoma, leukemia, brain tumor, epithelial cell-derived neoplasm (epithelial carcinoma), basal cell carcinoma, adenocarcinoma, lip cancer, oral cancer, esophageal cancer, gastrointestinal cancers such as small intestine cancer and stomach cancer, colon cancer, rectal cancer, liver cancer, bladder cancer, pancreatic cancer, ovarian cancer, cervical cancer, lung cancer, breast cancer, skin cancers such as squamous cell cancer and basal cell cancer, prostate cancer, and renal cell carcinoma, as well as metastases of these cancers, such as, for example, peritoneal disseminations caused by metastasis from digestive cancers such as stomach cancer, pancreatic cancer and colon cancer, and from ovarian cancer and ureteral cancer.

NIS-DPC as an active ingredient of the composition according to the present embodiment may be suspended in a solution capable of suspending it, such as, for example, a normal saline or a phosphate-buffered saline (PBS). Furthermore, the NIS-DPC suspension may comprise pharmaceutically acceptable additives.

When the composition according to the present embodiment is used for the treatment or diagnosis of cancer, the route of administration into a living body may be, for example, intratumoral (a cancer lesion or a vicinity thereof, for example, in an abdominal cavity when the treatment target is peritoneal dissemination) administration, or intravenous administration. In addition, the route of administration of the radionuclide administered in combination may be, for example, intratumoral (a cancer lesion or a vicinity thereof, for example, in an abdominal cavity when the treatment target is peritoneal dissemination) administration, intravenous administration, or oral administration.

NIS-DPC as an active ingredient of the composition according to the present embodiment may be provided in a frozen state. In this case, the frozen solution containing NIS-DPCs may be thawed several days, for example 10 days, before administration, and the NIS-DPCs may be then allowed to proliferate to the necessary number of cells before administration, or may be thawed immediately before administration and may be then administered without subjecting to a culture step.

The order of administration of the composition according to the present embodiment and the radionuclide is not particularly limited, and it is preferable to first administer the composition according to the present embodiment and then to administer the radionuclide. The interval between administrations of the composition according to the present embodiment and the radionuclide is not particularly limited, and it is preferably, for example, approximately 1 day to 7 days. In addition, the number of times of administration of the composition according to the present embodiment and the radionuclide may be once or multiple administrations. The administered dose of the composition according to the present embodiment and the radionuclide is not particularly limited, as long as it does not place an excessive burden on the living body and is an amount that can achieve a therapeutic effect or diagnostic purpose. The administered dose of the present composition and the radionuclide can be determined by the judgment of an expert such as a doctor.

The composition according to the present embodiment (a therapeutic composition and a diagnostic composition) may be provided in the form of a kit together with a usage manual that describes the administration method, etc. Each active ingredient (i.e., DPCs or NIS-DPCs, a radionuclide, etc.) of the composition included in the kit is supplied, separately, by a container made of a material that effectively maintains the activity of the active ingredient for a long period of time, does not allow the ingredients to adsorbed on the inside of the container, and does not deteriorate the ingredients. It is to be noted that the radionuclide must be supplied in a form suitable for preventing radiation leakage.

Moreover, the usage instructions of the present kit may be printed on a paper, etc., or may be stored on electromagnetically readable media such as CD-ROM or DVD-ROM, and may be then supplied.

A third embodiment relates to a method for treating a cancer, comprising administering the composition according to the present embodiment and the radionuclide to a patient (hereinafter also referred to as "the method for treating a cancer according to the present embodiment"). The radionuclide used in the present embodiment is preferably a nuclide that is incorporated by NIS-DPCs and emits α rays or β rays, which have high cell killing ability, and the radionuclide used herein may be, for example, ¹³¹I, ¹⁸⁸Re or ²¹¹At.

Herein, the term "treatment" means preventing or alleviating the progression and deterioration of the pathological conditions in a patient who has already been affected with a cancer, and it is a procedure for the purpose of preventing or alleviating the progression and deterioration of the cancer.

Moreover, the subjects of treatment are not limited to humans, and may be mammals other than humans, including: for example, mice, rats, dogs and cats; livestock animals such as bovines, horses and sheep; and primates such as monkeys, chimpanzees and gorillas. The subject of treatment is particularly preferably a human.

The method for treating a cancer according to the present embodiment comprises a step of administering NIS-DPCs and a step of administering a radionuclide. The interval between administrations of the NIS-DPCs and the radionuclide is not particularly limited, and it is preferably, for example, approximately 1 day to 7 days. The method of administering the radionuclide may be administration of the radionuclide to tumor tissues (cancer lesion) or tissues where tumor cells exist, intravenous administration, intraperitoneal administration, or oral administration. In addition, the number of times of administration of the NIS-MSCs and the radionuclide may be once or multiple administrations.

The method for treating a cancer according to the present embodiment may be performed in combination with other chemotherapy or radiation therapy, etc.

The cancer (malignant tumor/neoplasm) that is the target of the method for treating a cancer according to the present embodiment is not particularly limited, and examples thereof may include hepatocellular carcinoma, cholangiocarcinoma, renal cell carcinoma, squamous cell carcinoma, basal cell cancer, transitional cell carcinoma, glandular cancer, malignant gastrinoma, malignant melanoma, fibrosarcoma, myxosarcoma, liposarcoma, leiomyosarcoma, rhabdomyosarcoma, malignant teratoma, angiosarcoma, Kaposi's sarcoma, osteosarcoma, chondrosarcoma, lymphangiosarcoma, malignant meningioma, non-Hodgkin's lymphoma, Hodgkin's lymphoma, leukemia, brain tumor, epithelial cell-derived neoplasm (epithelial carcinoma), basal cell carcinoma, adenocarcinoma, lip cancer, oral cancer, esophageal cancer, gastrointestinal cancers such as small intestine cancer and stomach cancer, colon cancer, rectal cancer, liver cancer, bladder cancer, pancreatic cancer, ovarian cancer, cervical cancer, lung cancer, breast cancer, skin cancers such as squamous cell cancer and basal cell cancer, prostate cancer, and renal cell carcinoma, as well as metastases of these cancers, such as, for example, peritoneal disseminations caused by metastasis from digestive cancers such as stomach cancer, pancreatic cancer and colon cancer, and from ovarian cancer and ureteral cancer.

A fourth embodiment relates to a method for diagnosing a cancer, comprising administering the composition according to the present embodiment and the radionuclide to a subject (hereinafter also referred to as "the method for diagnosing a cancer according to the present embodiment").

The method for diagnosing a cancer according to the present embodiment comprises a step of administering NIS-DPCs to a subject, a step of administering to the subject, a radionuclide that can be incorporated by NIS-DPCs, and a step of identifying a site in the living body where the radionuclide accumulates. The radionuclide used in the method for diagnosing a cancer according to the present embodiment is preferably a nuclide that can be incorporated by NIS-DPCs and can be detected by a device capable of identifying the location of the radionuclide, such as, for example SPECT (Single Photon Emission Computed Tomography) or PET (Positron Emission Tomography), and for example, a nuclide that emits γ rays is preferable, and for example, such as ¹²³I, ¹²⁵I, ¹²⁴I or ^{99m}Tc, etc. is preferable.

When an English translation of the present description includes terms with the articles "a," "an," and "the," these terms include not only single items but also multiple items, unless otherwise clearly specified from the context that it is not the case.

Hereinafter, the present invention will be further described in the following examples. However, these examples are only illustrative examples of the embodiments of the present invention, and thus, are not intended to limit the scope of the present invention.

### Examples

### [Example 1] Preparation of NIS-DPCs

### 1. Preparation of lentivirus and quality test

### 1.1. Construction of pLVSIN-EF1α-hNIS-Pur

The self-inactivating (SIN)-type lentivirus vector pLVSIN-EF1α-hNIS-Pur was prepared by inserting a nucleic acid sequence encoding human NIS (human NIS, hereafter referred to as "hNIS", SEQ ID No: 2, NCBI Accession Number: NM_000453.3) into the multicloning site of a pLVSIN-EF1α-Pur vector (Takara Bio. Inc.). Specifically, the DNA encoding hNIS was artificially synthesized with a Not I site on the 5' side and a BamH I site on the 3' side, and was then treated with the corresponding restriction enzymes. Next, the pLVSIN-EF1α-Pur vector was cleaved with the restriction enzymes Not I and BamH I to linearize the vector, and the resulting vector was then ligated to hNIS treated with restriction enzymes, using high Ver.2 (TOYOBO), so as to prepare pLVSIN-EF1α-hNIS-Pur.

### 1.2. Lentivirus packaging

In lentivirus packaging, in addition to the SIN-type lentivirus vector (pLVSIN-EF1α-hNIS-Pur) prepared in 1.1 above, the Lentiviral High Titer Packaging Mix (hereinafter referred to as "Packaging Mix") (Takara Bio, Inc.), which is a mixture of plasmids containing the components necessary for preparing the lentivirus vector (HIV-1-derived lentivirus proteins Gag, Pol, Tat and Rev, and a VSV-G envelope protein), was used, and the standard protocols were partially modified for preparation. Specifically, 4 x 10⁶ cells were seeded on a 10 cm dish treated with Poly-L-Lysine, and the day after the seeding, 7 µL of Packaging Mix and 11 µL of pLVSIN-EF1α-hNIS-Pur adjusted to 0.5 µg/µL were added to 1.5 mL of OptiMEM I Reduced Serum Medium (Gibco) to prepare a DNA Mix. Thereafter, Fugene HD (Promega) or TransIT-Lenti Transfection Reagent (Takara Bio, Inc.) was added to the prepared DNA Mix, and transfection was performed. The cells were then cultured at 37°C in a 5% CO₂ incubator. Approximately 24 hours after the transfection, the supernatant was aspirated and removed, and the medium was replaced with 10 mL of DMEM medium, and thereafter, the cells were cultured at 37°C in a 5% CO₂ incubator for approximately 24 hours. Thereafter, the culture supernatant was recovered, and was then centrifuged at 500 x G for 10 minutes. The resultant was passed through a 0.45 µm filter, and the supernatant was then dispensed into a microtube, etc. with a pipette, and was preserved at -80°C. In addition, a portion of the recovered culture supernatant was concentrated using a Lenti-X Concentrator (Takara Bio, Inc.).

### 1.3. Titer measurement of lentivirus

The prepared lentivirus was measured in terms of three items, namely, p24 protein amount (ELISA), viral genome amount (qPCR), and biological infectivity titer (flow cytometer). The p24 protein amount was measured using the Lenti-X p24 Rapid Titer Kit (Takara Bio, Inc.), and the viral genome amount was measured using the Lenti-X qRT-PCR Titration Kit (Takara Bio, Inc.). The biological infectivity titer was calculated by infecting cultured HEK293T cells with the lentivirus and measuring the hNIS-positive percentage by flow cytometry two days after the infection.

The various titers of the prepared lentivirus were approximately 20 ng/mL to 200 ng/mL (p24 protein amount), 1 x 10⁷ VG/mL to 1 x 10⁹ VG/mL (viral genome amount), and approximately 1 x 10⁶ TU/mL to 6 x 10⁶ TU/mL (biological infectious titer). respectively. The titers of the concentrated virus were approximately 200 ng/mL to 400 ng/mL (p24 protein amount), 1 x 10⁸ VG/mL to 6 x 10⁹ VG/mL (viral genome amount), and approximately 1 x 10⁷ TU/mL to 3 x 10⁷ TU/mL, respectively. The concentrated solution was used as an hNIS-LV stock solution.

### 2. Studies of the method of modifying DPC functions using lentivirus

Studies were conducted regarding infection conditions that would enable functional modification of DPCs using lentivirus, without impairing the properties of the DPCs.

### 2.1. Studies of conditions for infection of DPCs with lentivirus

### 2.1.1. Studies of auxiliary reagents

Polybrene and protamine, which are commonly known as auxiliary reagents for promoting infection of cells with lentivirus, were compared and were evaluated, regarding the influence on cell proliferation after the infection and on the introduced hNIS-positive percentage.

### 2.1.1.1. Methods

The day after the seeding of DPCs at 20,000 cells/cm², an hNIS-LV stock solution was added to the cells to approximately 90 µL/cm². At that time, to the virus stock solution, a polybrene solution with a concentration of 8 µg/mL after addition, or a protamine sulfate solution with a concentration of 100 µg/mL after addition was added. The hNIS-LV stock solution was removed the day after the addition, and an equal amount of DMEM medium was added. After that, the culture was continued, and such continuous culture was performed for 22 days after the infection. Then, doubling days and accumulated PDL (Population doubling level) values were calculated and compared.

In addition, the influence of the number of passages on the hNIS-positive percentage was compared (the hNIS-positive percentage was confirmed up to the 6th passage).

### 2.1.1.2. Results

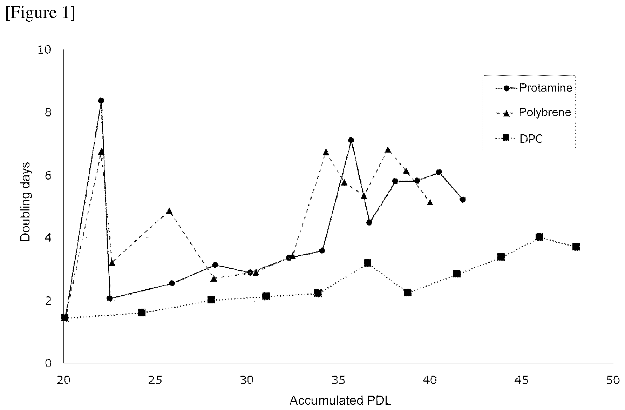

A transition in the doubling days of DPCs after lentivirus infection is shown in Figure 1. The doubling days immediately after the virus infection were 6.75 days in a group infected with lentivirus using polybrene and 8.36 days in a group infected with lentivirus using protamine. Compared to 1.6 days for DPCs measured in parallel as a control, both of the above values were higher, and a tendency for proliferation to temporarily decrease was observed. After that, as a result that the culture was continued, although proliferation was lower than that of DPCs, proliferation was considered to be able to be maintained for about six passages after the infection. In addition, the use of protamine as an auxiliary reagent was considered to suppress a decrease in proliferation, rather than polybrene.

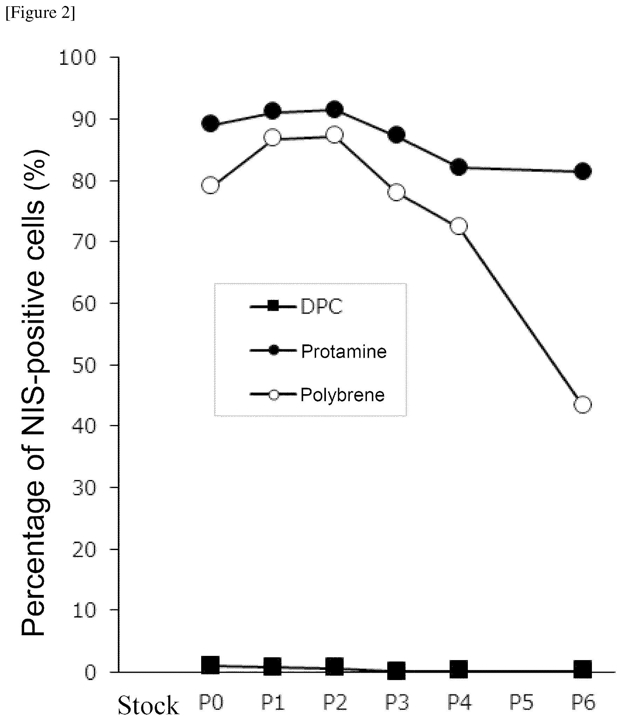

Moreover, a transition in the hNIS-positive percentage upon the continuous culture of DPCs is shown in Figure 2. Uninfected DPCs were also measured as a control. The hNIS-positive percentage immediately after initiation of the culture was 89.0% in a group infected with the virus using protamine and was 79.0% in a group infected with the virus using polybrene. Thus, DPCs infected with the virus using protamine had a higher hNIS-positive percentage. Thereafter, the culture was continued, and the hNIS-positive percentage was confirmed upon passage. As a result, the positive percentage tended to gradually decrease, but it was considered that a high positive percentage could be maintained for 2 or 3 passages. In addition, it was found that protamine, which had a higher positive percentage, was able to maintain the positive percentage, compared to the infection using polybrene. It was assumed that when the positive percentage is low, the positive percentage would decrease as the number of uninfected cells gradually increases. Therefore, it was considered that the hNIS-positive percentage after the infection should be aimed to be 90% or more.

From the above, it was considered that the use of protamine as an auxiliary reagent during the infection would enable more efficient gene introduction by lentivirus, and that the hNIS-positive percentage could be maintained even after subculture. Thus, protamine was used as an auxiliary reagent during infection of DPCs with lentivirus.

### 2.1.2. Studies of the titer at the time of infection

The virus titer at the time of infection (multiple of infection: MOI) required upon addition of the lentivirus to DPCs was studied for optimization.

### 2.1.2.1. Methods

The hNIS-LV stock solution prepared in 1.3 above was used. The hNIS-LV stock solution was added on the day after DPCs had been added at 20,000 cells/cm². At that time, a 100 µg/mL protamine sulfate solution was added to the hNIS-LV stock solution as a reagent for promoting the virus infection of the cells. The hNIS-LV stock solution was removed the day after the addition, and an equal amount of DMEM medium was added. Three lots of hNIS-LV stock solutions with different titers were used, and two-fold dilution from the stock solution was repeated four times, and DPCs were infected with 5 stages of virus amounts (see Table 1).

**[Table 1]**

| | Infectivity titer (x 10⁶ TU/mL) | Multiplicity of infection (MOI)* when stock solution is used |
|---|---|---|
| LV① | 3.05 | MOI 14 |
| LV② | 2.78 | MOI 12 |
| LV③ | 5.83 | MOI 5 |

| | | |
|---|---|---|
| *Multiplicity of infection (MOI): An indicator that quantifies the amount of virus added per cell. MOI 10 means that the amount of virus that is 10 times the number of cells is added. | | |

The cells were passaged on the 4th day after the lentivirus infection, and were then cultured using DMEM supplemented with puromycin to 1 µg/mL. Thereafter, at the timing when the cells under individual conditions became confluent, they were passaged again (were cultured in puromycin-free DMEM), and the hNIS-positive percentage of the cells harvested after a total of two passages was measured by flow cytometry analysis.

### 2.1.2.2. Results

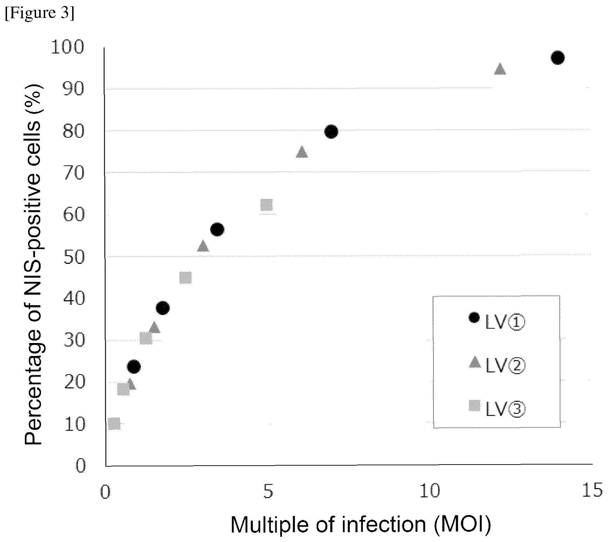

The relationship between the MOI at the time of infection and the ratio of hNIS-positive cells when the cells were harvested after two passages is shown in Figure 3. A strong correlation was observed between the MOI and the hNIS-positive percentage, and it could be confirmed that the hNIS-positive percentage increased as the MOI of the virus to be added increased. In addition, it was suggested that a positive percentage of 80% could be achieved when the MOI exceeded 8, and that when the MOI became 15, the target gene hNIS could be introduced into nearly 100% of the cells. In the culture after the lentivirus addition, selection was performed to obtain only positive cells using antibiotics, but the hNIS-positive percentage did not increase as expected. Therefore, it was considered that the addition of a virus amount (=MOI) at the time of lentivirus infection that can ensure a sufficient positive percentage is important. Considering the results of the previous studies and the currently assumed hNIS-DPCs production process, the hNIS-positive percentage needs to target at around 90% at the time of infection. Therefore, the target value of MOI of the lentivirus added to the DPCs was set to be 10 or more.

### 3. Preparation of hNIS-introduced DPCs

The hNIS-LV stock solution prepared in 1.3 above was added to DPCs to prepare hNIS-introduced DPCs (hNIS-DPCs). The infection conditions were those as found in the previous studies.

### 3.1. Preparation of hNIS-DPCs

Preparation of hNIS-DPCs was carried out roughly as follows.

The day after DPCs had been seeded at 20,000 cells/cm², the hNIS-LV stock solution was added at an MOI of approximately 10. At that time, a protamine sulfate solution was added to the virus stock solution as a reagent for promoting the virus infection of the DPCs to a concentration of 100 µg/mL after the addition. The hNIS-LV stock solution was removed the day after the addition, and an equal amount of DMEM medium was added.

### 3.1.1. Culture of DPCs

The frozen DPCs were thawed, and the cells were then seeded on a 10 cm dish to 20,000 cells/cm². Gene introduction by a lentivirus vector was carried out on the seeded cells using a protamine sulfate solution according to the conditions shown in 2.1 above, and on the 4th day of the culture, the cells were harvested and were then seeded at 7,000 cells/cm² in a T225 flask. On the 4th day of the culture, the medium was replaced with DMEM supplemented with 1 µg/mL puromycin.

On the 6th day of the culture, the cells were harvested and were then seeded at 8,000 cells/cm² in two T1000 flasks. On the 6th day of the culture, the cells were harvested and were then dispensed into vials at 3 x 10⁶ cells per vial, and were stored in a frozen state.

### 4. Confirmation of quality of hNIS-DPCs

The quality of the hNIS-DPCs prepared in 3.1 above was evaluated.

### 4.1. Methods

The evaluation items were (1) doubling days at the time of freezing, (2) PDL (the number of cell divisions) and accumulated PDL, (3) the percentage of hNIS-positive cells, and (4) the relationship between the percentage of hNIS-positive cells and the amount of iodine uptake.

Doubling days and PDL were calculated using the following calculation formulae.

### [Doubling days]

The number of culture days × Log10 2/(Log10 (the number of harvested cells) - Log10 (the number of seeded cells))

### [PDL (the number of cell divisions)]

(Log10 (the number of harvested cells) - Log10 (the number of seeded cells)) × Log2 10

The presence of hNIS-positive cells was confirmed by flow cytometry analysis. The harvested cells were suspended in a blocking agent prepared by adding 30% BSA (Fujifilm Wako Pure Chemical Industries, Ltd.) into DPBS (Gibco) to result in 3% BSA, and the obtained suspension was then left at rest at room temperature for 30 minutes or more, or at 4°C until the next day. Thereafter, Stain Buffer (BD) was added to suspension, and the obtained mixture was then washed by centrifugation. The cells were suspended in an antibody solution prepared by adding an hNIS antibody (Human SLC5A5C2 antibody (R&D)) to Stain Buffer to an antibody amount of 0.25 µg to 0.5 µg per 1 x 10⁶ cells, and the obtained suspension was then left at rest at room temperature for 30 minutes or more to perform a primary antibody reaction. After that, the cells were washed three times by centrifugation with Stain Buffer, and were then suspended in an antibody solution prepared by 400-fold diluting Alexa Fluor 488 goat anti Mouse IgG antibody (Invitrogen) with Stain Buffer. The suspension was left at rest at room temperature under light-shielded conditions for 30 minutes or more to perform a secondary antibody reaction. Thereafter, the resulting cells product were washed three times by centrifugation with Stain Buffer, and flow cytometry measurement was then performed. The percentage of hNIS-positive cells was calculated by measuring a sample prepared by treating DPCs according to the above-described procedures, and adjusting the gate position so that the cell population within the hNIS-positive gate in the FITC-A histogram was approximately 0.1 to 1%.

The amount of iodine uptake was measured using the method described in 4.3.1 below.

### 4.2. Results

The doubling days of the prepared hNIS-DPCs were 2.1 days, the PDL was 2.9, the accumulated PDL was 24.0, and the percentage of hNIS-positive cells was 94.1%.

Since the doubling days of the non-transfected DPCs were around 1.5 to 2.0, the influence of lentivirus-mediated hNIS gene transduction on cell proliferation was considered to be minor.

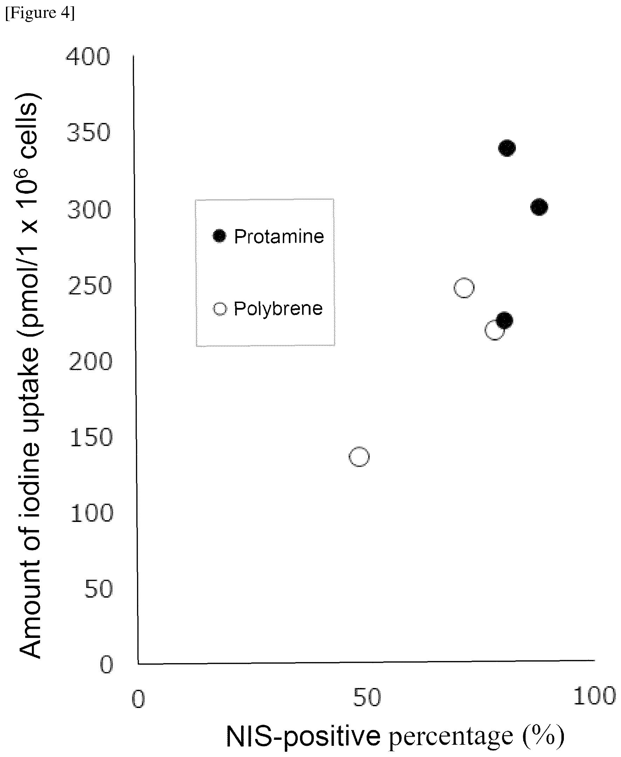

In addition, the relationship between the amount of iodine uptake and the percentage of hNIS-positive cells is shown in Figure 4. In both cases of virus infection using protamine and polybrene, a linear positive correlation was observed between the percentage of hNIS-positive cells and the amount of iodine uptake, so that it could be confirmed that the NIS expressed in the DPCs normally functioned.

### 4.3. Quality comparison with hNIS-mouse MSCs

The quality of cells in which hNIS was introduced into mouse-derived MSCs prepared separately (hereinafter referred to as "hNIS-mouse MSCs") was compared with the quality of the hNIS-DPCs prepared by the present method. The comparison was performed based on the amount of iodine uptake via a transporter, which is thought to be important for exhibiting medicinal effects.

### 4.3.1. Methods

The hNIS-mouse MSCs or the hNIS-DPCs were seeded at 50,000 cells/cm² on a 96-well plate or a 48-well plate, and culture was then initiated at 37°C in 5% CO². After washing the cells twice with 10 mM HEPES-HBSS on the day after initiation of the culture, an NaI solution prepared by adjusting NaI (FUJIFILM Wako Pure Chemical Corporation) with 10 mM HEPES-HBSS to 5 µM was added to the resulting cells, and the thus obtained mixture was then incubated at 37.0°C and in 5.0% CO₂. After 4 hours had passed, the reaction mixture was washed twice with HEPES-HBSS, and water for injection was then added thereto. The obtained mixture was left at rest for one day at maximum until NaI was eluted from the cells, and the cell eluate was then recovered. Thereafter, the iodine concentration in the eluate was measured using a Non-Radioactive Iodide Assay Kit (Bertin Pharma) to calculate the amount of iodine uptake into the cells.

### 4.3.2. Results

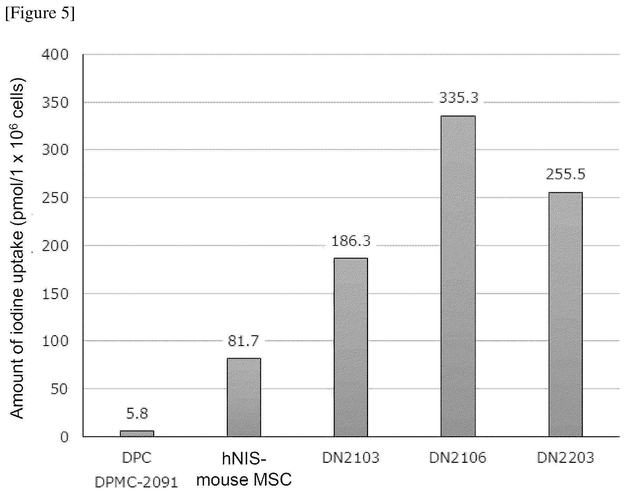

The amount of iodine uptake was calculated as an amount per 1 x 10⁶ cells. Among the hNIS-DPCs prepared in 4.1.2 above, three lots of cells were measured. As a control, intact DPCs without hNIS introduction were also measured. The results are shown in Figure 5. Almost no iodine uptake was observed in the intact DPCs without hNIS introduction. On the other hand, it was confirmed that all lots of hNIS-introduced DPCs incorporated a larger amount of iodine than hNIS- mouse MSCs. Therefore, it was considered that the hNIS-DPCs prepared using lentivirus in the present study can maintain their iodine transporter function and can incorporate a large amount of iodine therein depending on the high positive percentage.

### [Example 2] In vivo test of hNIS-DPCs

### 1. Preparation of peritoneal dissemination models

All animal experiments were approved by the University of Tokyo Animal Experiment Committee and were performed in accordance with the guidelines.

Five-week-old C57BL/6 mice (Charles River Laboratories Japan, Inc.) were intraperitoneally administered with the transplantable mouse stomach cancer cell line YTN16 (1 x 10⁷ cells/mouse) to form peritoneal dissemination. YTN16 is a cell line established by the present inventors (Non Patent Literature 5). One to three weeks after administration of YTN16, it was confirmed that peritoneal dissemination was formed on the peritoneum that could be confirmed with naked eyes.

### 2. Evaluation of migration and accumulation of DPCs in the vicinity of cancer tissues by intraperitoneal and intravenous administration of the DPCs

### 2.1. Methods

### 2.1.1. Administration of intact DPCs

After thawing, culturing and expanding DPCs, the harvested cells were frozen (using two types of cryoprotectants, TC protector and Cell Banker). Before administration to animals, DPCs were thawed and precultured, and a necessary amount of cells was suspended in a medium. The number of cells to be administered was 3 doses, namely, 5 x 10⁶ cells/body, 1 x 10⁶ cells/body, and 0.5 x 10⁶ cells/body for both intraperitoneal and intravenous administration. Each number of cells was administered to three peritoneal dissemination models (i.e., for each of intravenous and intraperitoneal administration, 9 peritoneal dissemination mouse models (3 doses x 3 mice) were used). DPCs were administered on the 15th day (Day 15), the 17th day (Day 17), and the 19th day (Day 19), counting the day of YTN16 administration as Day 1.

### 2.1.2. Method of evaluating migration and accumulation of DPCs

Cell migration and accumulation were evaluated by immunostaining tissue sections. Mice were euthanized on the 21st day (Day 21) after YTN16 administration, and the peritoneum with peritoneal dissemination was excised. The peritoneum was pinned out on a rubber plate and was then fixed overnight in 4% neutral buffered paraformaldehyde. After fixing, the peritoneum was transferred into a 50 mL Falcon tube, and it was washed with PBS on a shaker for 20 minutes x 3 times or more, and was further washed overnight with PBS. Thereafter, paraffin blocks were prepared by outsourcing to Advantec Co., Ltd. Sections were prepared from the prepared paraffin blocks, and were then immunostained using an anti-human Lamin B1 antibody (Anti-human Lamin B1: HS-404 017 (Rat monoclonal purified IgG), HistoSure). Thereafter, the presence or absence of stained cells was evaluated.

### 2.2. Results

When the number of cells administered was evaluated regarding each administration route, three animals died in a group administered intravenously at 5 x 10⁶ cells/body, and one animal died in a group administered intravenously at 1 x 10⁶ cells/body. On the other hand, no deaths were observed after intraperitoneal administration.

Tissue sections prepared from peritoneal tissues were confirmed to see if nonspecific staining was observed in the cancerous area. As a result, no nonspecific staining was observed in peritoneal tissues from normal mice and in peritoneal tissues with peritoneal dissemination, when DPCs were not administered. Therefore, it was determined that the present antibody (anti-human Lamin B1 antibody) can be used to appropriately detect the presence or absence of human cells in peritoneal tissues.

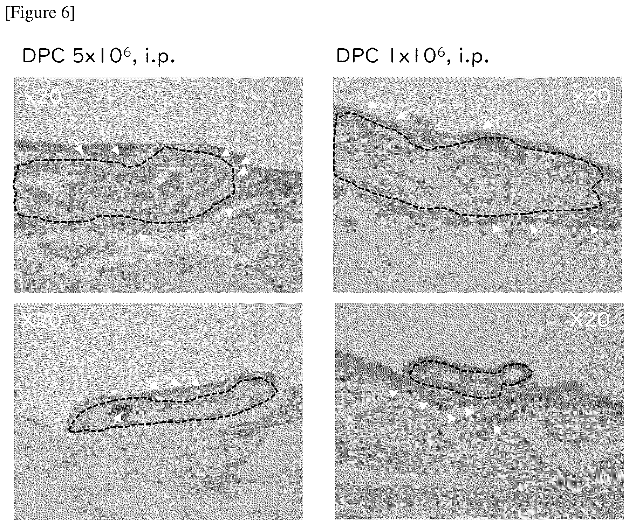

The results of representative immunostaining of peritoneal tissues in mice intraperitoneally administered with cells are shown in Figure 6. Staining images suggesting the presence of the administered DPCs were observed in the peritoneal tissues of all mice, and accumulation of the DPCs was observed in a form surrounding the periphery of the cancer tissues (the area enclosed with the dashed line in the figure is the cancer tissues). In addition, a difference was observed in the degree of staining depending on the number of cells, and when the cells were administered in a cell count of 1 x 10⁶ cells/body or more, there did not seem to be a significant difference in the staining images.

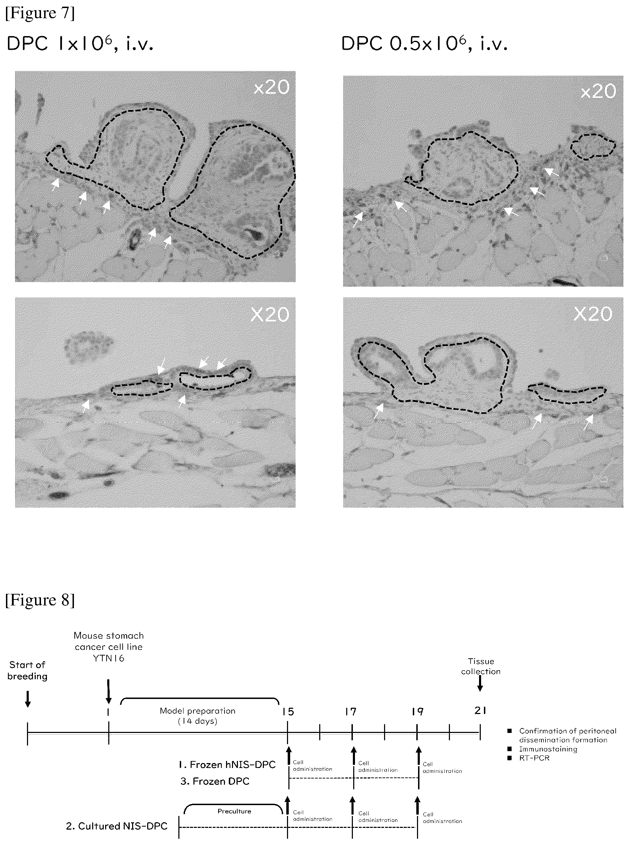

Representative results in mice intravenously administered with the cells are shown in Figure 7. Since all mice intravenously administered with the cells in a cell count of 5 x 10⁶ cells/body died, only the results of mice administered with the cells in a cell count of 1 x 10⁶ cells/body and 0.5 x 10⁶ cells/body are shown. Since specific staining derived from DPCs was observed even in mice administered intravenously, as in mice administered intraperitoneally, it was suggested that DPCs migrate to cancer tissues even when the cells are administered via the present route of administration. The number of cells accumulating by intravenous administration seemed to be less than that by intraperitoneal administration.

From the aforementioned results, it was suggested that DPCs migrate to cancer tissues not only via intraperitoneal administration but also via intravenous administration. Based on the thus obtained results, the upper limit value of the number of cells administered in future studies was set to be 1 x 10⁶ cells/body.

### 3. Evaluation of migration and accumulation of hNIS-DPCs in the vicinity of cancer tissues by intraperitoneal administration of the hNIS-DPCs

From the results of the previous studies, it could be confirmed that DPCs migrate in the vicinity of cancer tissues. Thus, whether a similar tendency was observed in DPCs into which a human sodium iodine symporter (NIS) had been introduced using lentivirus (hereinafter referred to as "hNIS-DPC") was evaluated.

### 3.1. Evaluation method

Evaluation was performed by the same method as that described in 2 above. Figure 8 shows the experimental schedule. In the present evaluation, two types of NIS-DPCs, namely, cells that were pre-cultured before administration (cultured products; Cultured) and frozen cells (frozen products; Frozen) were used, and the evaluation was performed by two routes of administration, namely, intraperitoneal administration and intravenous administration. The number of cells administered was set to be the following: intravenous administration (1 x 10⁶ cells/body and 0.5 x 10⁶ cells/body) and intraperitoneal administration (1 x 10⁶ cells/body). In addition, DPCs (frozen products only) were set as controls, and were evaluated by intravenous administration (0.5 x 10⁶ cells /bod) and intraperitoneal administration (1 x 10⁶ cells/body) (a total of 24 mice). Detection of migrated cells was performed by PCR and immunostaining.

Evaluation by PCR was performed as follows. Peritoneal tissues were collected from euthanized mice, and were homogenized, and then, RNA was extracted, and was reverse transcribed, and then, PCR was performed using the obtained cDNA as a template. Three tissue samples were collected from each individual. Cell accumulation was evaluated based on the expression of the human gene GAPDH. Mouse GAPDH was used as a control.

PCR conditions were as follows.
Template amount
   1/50 of the amount of cDNA produced from 1 µg of RNA
Thermal cycler settings
   94°C 5 min, (94°C 30 sec, 60°C 30 sec, 72°C 30 sec) x 40 cycles, 72°C 30 sec
Primers used
   Mouse GAPDH; Amplified size: 177 bp, human GAPDH; Amplified size: 203 bp

### 3.2. Results

### 3.2.1. Evaluation by PCR

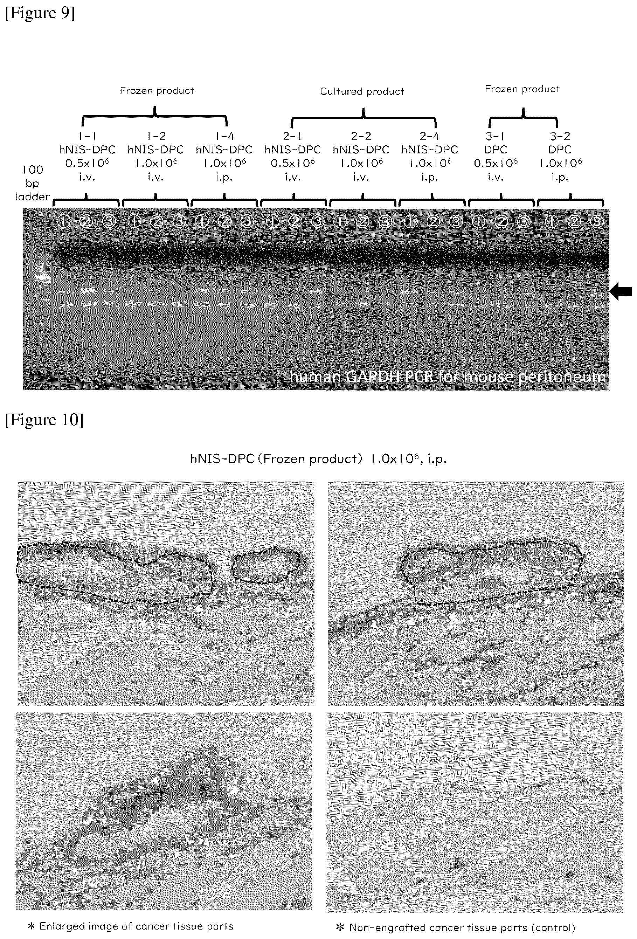

The results obtained by evaluation by PCR are shown in Figure 9. In the group administered with DPCs (frozen products), the expression of human GAPDH was detected in both intravenous and intraperitoneal administration. The detection varied depending on individuals, but this was presumed to be due to the location of the collected peritoneal tissues. Also in the group administered with hNIS-DPCs, whether the hNIS-DPCs are frozen products or cultured products, the expression of human GAPDH could be confirmed, as with DPCs. Moreover, the expression was observed regardless of the route of administration, and there was no significant difference in any doses.

### 3.2.2. Evaluation by immunostaining

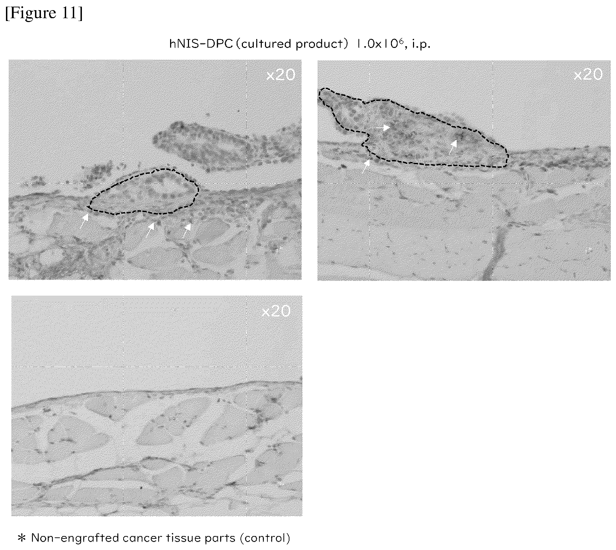

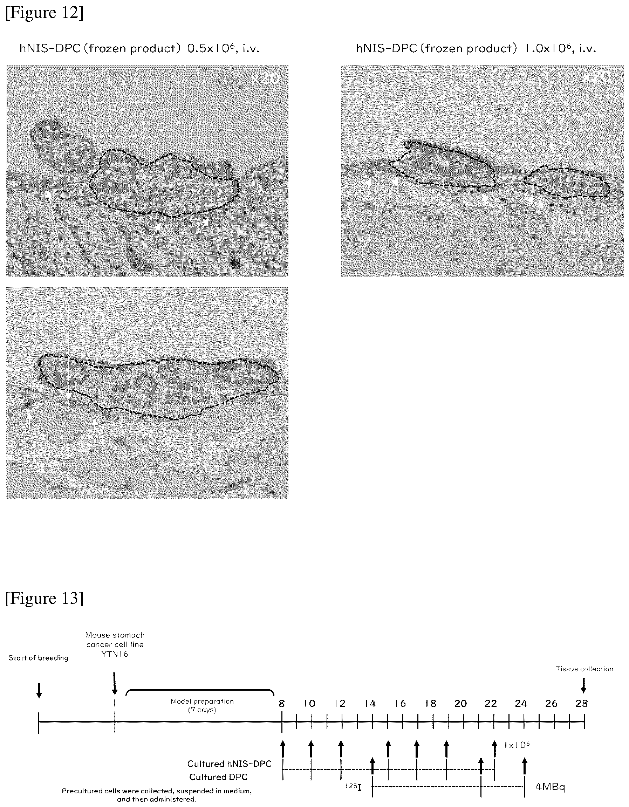

Representative immunostaining results obtained when the frozen hNIS-DPCs were thawed and were intraperitoneally administered without preculture are shown in Figure 10, and representative immunostaining results obtained when the hNIS-DPCs were intraperitoneally administered after preculture are shown in Figure 11. In addition, representative immunostaining results obtained when frozen hNIS-DPC were thawed and were intravenously administered without preculture are shown in Figure 12. In individuals administered with hNIS-DPCs, staining derived from human cells was observed in the vicinity of the peritoneal tissues, regardless of whether the samples were frozen products or cultured products. When comparing intravenous administration with intraperitoneal administration, it appeared that more cells accumulated in the case of intraperitoneal administration (for example, compare the sites indicated with the arrows in Figure 10, Figure 11, and Figure 12).

### 3.2.3. Summary

From the results of PCR and immunostaining, it was suggested that cells accumulated and infiltrated in the vicinity of the cancer tissues even in the case of hNIS-DPCs whose functions were modified by lentivirus, regardless of the route of administration. Although it is not quantitative, the amount of accumulating cells seemed to be larger in the case of intraperitoneal administration than in the case of intravenous administration. In addition, in the case of intraperitoneal administration, there seemed to be no significant difference in the degree of accumulation between the frozen products of hNIS-DPCs and the cultured products of hNIS-DPCs. Taking into consideration that clinical trials are to be conducted, administering cells into the abdominal cavity does not impose a large burden on patients, since a port for drug administration has been installed in many cases. Moreover, intraperitoneal administration allows cells to be administered locally and is expected to have higher medicinal effects. Accordingly, it has been decided to select intraperitoneal administration as a first choice of administration method in future studies.

### 4. Evaluation of migration and accumulation of hNIS-DPCs in the vicinity of cancer tissues using ¹²⁵I

Whether the administered hNIS-DPCs accumulate in cancer tissues, and whether hNIS expressed in DPCs can incorporate a radionuclide, were evaluated. As a radionuclide, ¹²⁵I, which emits γ rays, was used.

### 4.1. Methods

Evaluation was performed by the same method as that described in 2 above.

For the purpose of shortening the evaluation period, the model preparation period after administration of YTN16 cells was changed from 14 days to 7 days. In order to suppress accumulation of ¹²⁵I in the thyroid gland, 2 µg of L-T4/d levothyroxine (thyroid hormone: Levothyroxine) was administered from the 4th day after YTN16 transplantation. The cells used in the test were only cells (culture products) that had been precultured before administration, and the administration route was intraperitoneal administration, and the number of cells administered was set to be 1 x 10⁶ cells/body. In addition, DPCs (also culture products) were used as controls. The experimental schedule is shown in Figure 13. Two groups, namely, one hNIS-DPCs administration group consisting of two mice and one DPCs administration group consisting of one mouse, were used as a pair, and the cells were administered thereto divided over three times. The number of cell administrations was 7 in total, and ¹²⁵I was administered three times (orally, 4 MBq). The day of YTN16 administration was counted as the first day, and the tissues were collected on Day 28. The γ radiation dose in each tissue was measured using a gamma counter to evaluate cell migration into individual tissues and the presence or absence of incorporation of the radioactive iodine ¹²⁵I. The γ radiation dose was measured in the following target tissues: peritoneum, liver, stomach, intestine, colon, pancreas, spleen, kidney, heart, lung, brain, ovary, thyroid gland, submandibular gland, and bone.

### 4.2. Results

### 4.2.1. Results of the γ radiation dose in each tissue

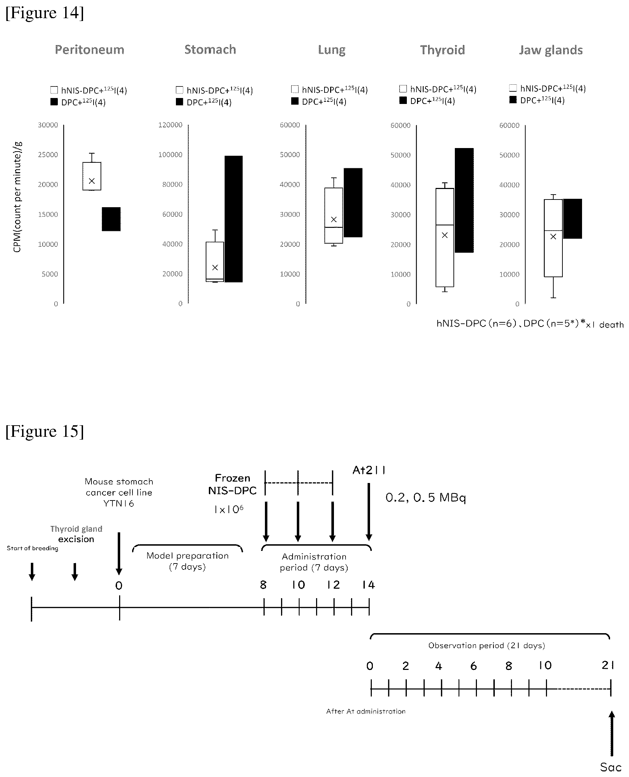

The results of the measurement of the γ radiation dose in major organs are shown in Figure 14. The γ radiation dose in peritoneal tissues was higher in hNIS-DPCs than in individuals administered with DPCs. Accordingly, it was suggested that the hNIS-introduced DPCs is localized in peritoneal tissues and incorporates the radioactive iodine.

In other tissues, the amount of the radioactive iodine accumulated was large in the stomach, lung, thyroid gland, and submandibular gland. Since, in mice, the expression level of NIS is high in the order of stomach, thyroid gland, submandibular gland, colon, testis and lung, it was considered that the amount of the radioactive iodine accumulated increases with the expression level of NIS. In addition, accumulation of the radioactive iodine in the lung was thought to be due to the influence by accumulation in the bronchi. Moreover, because ¹²⁵I was orally administered (intragastric administration) rather than intraperitoneal administration, it was considered that radiation remained in the digestive tract and remained in the stomach in a large amount, which may have led to a variation in individuals.

### 4.2.2. Summary

From the aforementioned results, it was suggested that hNIS-DPCs administered to the peritoneal dissemination models accumulate in the vicinity of the cancer tissues, and that the administered ¹²⁵I is taken up via NIS expressed in DPC. Therefore, it could be confirmed that it is possible to accumulate the cells in the vicinity of the cancer tissues by using NIS-DPCs as radiation delivery carriers.

### 5. Evaluation of the influence of ²¹¹At on cancer tissues when ²¹¹At is used as a radionuclide (1)

²¹¹At is a nuclide that emits α rays. The α rays are heavy ion beams that cleave the double strands of DNA and cause significant damage to cancer cells, and these effects are thought to be exhibited independently from the cell cycle, compared to β rays. In addition, since the α rays are heavy ion beams, they are considered to be advantageous in many respects in that the flight distance is short and little damage is given to surrounding normal cells other than cancer cells, in that the α rays have few side effects because they penetrate only several cells, in that their half-life is short, assuming that the α rays are administered to patients, in that the α rays are excreted by being mixed with urine and feces, etc., and in that there is no risk of radiation exposure from the patients to people around them. Therefore, the α rays do not require long-term isolation hospitalization, differing from ¹³¹I, which is currently used in the treatment of thyroid cancer, and it is considered to be much superior to conventional treatment methods using β-nuclides.

Thus, next, in order to examine the possibility of peritoneal dissemination treatment using ²¹¹At, using hNIS-DPCs as delivery carriers for ²¹¹At, the cytotoxicity of ²¹¹At against peritoneal dissemination was evaluated using mouse peritoneal dissemination models.

In order to optimize the dose of ²¹¹At administered, its cytotoxicity was evaluated with target doses of 0.2MBq and 0.5MBq, based on the doses that had previously been administered to humans.

### 5.1. Methods

The experimental schedule is shown in Figure 15. With regard to group configuration, 5 groups were established: (1) an untreated group (n = 10), (2) a ²¹¹At (0.2 MBq/body) administration group (n = 10), (3) an hNIS-DPC/²¹¹At (0.2 MBq/body) administration group (n = 10), (4) a ²¹¹At (0.5 MBq/body) administration group (n = 10), and (5) an hNIS-DPC/²¹¹At (0.5 MBq/body) administration group (n = 10). Frozen cells were thawed to prepare cells to be administered, and the number of cells administered was set to be 1 x 10⁶ cells/body. After three times of cell administrations, ²¹¹At was administered once. Regarding the route of administration, both the cells and ²¹¹At were intraperitoneally administered. After ²¹¹At administration, the mice were observed for 21 days, were then euthanized, and were then evaluated. Evaluation was performed by scoring the degree of peritoneal dissemination based on visual observation into 5 levels (4: large tumor particles present over the entire surface, 3: some tumor particles present but not over the entire surface, 2: small tumor particles clearly present, 1: only a few tumor particles can be confirmed by close observation, 0: no tumor particles present). A score of 4 was given if a large amount of peritoneal dissemination remained, and a score of 0 was given if such peritoneal dissemination had almost disappeared.

Moreover, in order to quantify the amount of cancer cells remaining in the peritoneal tissues, the Fgfr4 gene, which is specifically expressed in YTN16, was measured by qPCR, and cytotoxicity was quantitatively evaluated. Specifically, tissues were collected from the peritoneum and were homogenized, and RNA was extracted and was then reverse transcribed. Using the obtained cDNA as a template, qPCR was performed. Two tissue samples were collected from one individual. Mouse Gapdh was used as a control. For evaluation, the measured value was calculated as a relative value when the expression level of the Fgfr4 gene in the untreated group was set as 1.

PCR conditions were as follows.
Template amount
   1/50 of the amount of cDNA produced from 1 µg of RNA
Settings of quantitative thermal cycler settings
   94°C 10 min, (95°C 10 sec, 60°C 30 sec, 72°C 15 sec) x 40 cycles
Primers used
   Mouse Fgfr4; Amplified size: 219 bp, mouse Gapdg; Amplified size: 177 bp

### 5.2. Results

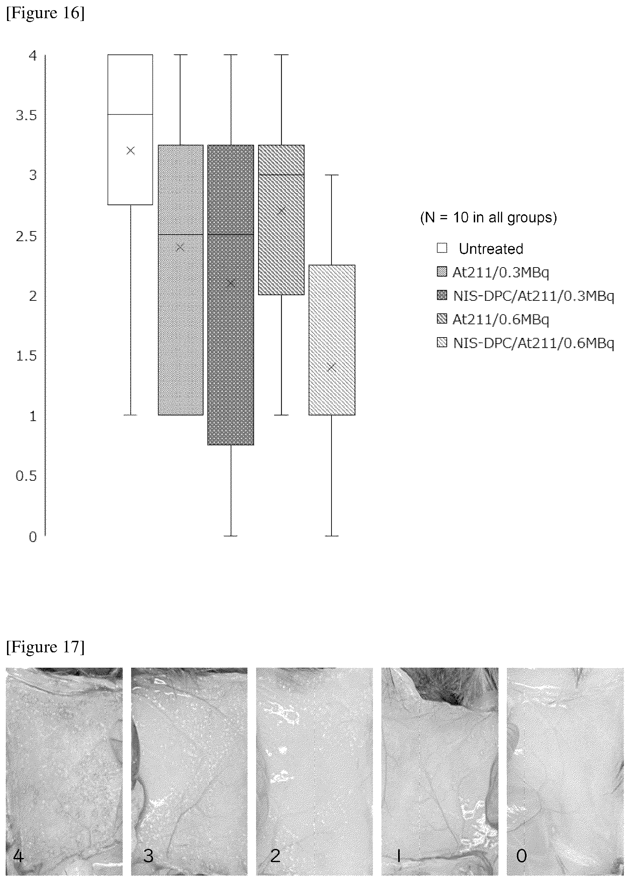

The evaluation results of the scoring by visual observation are shown in Figure 16. In addition, representative images at the time of the visual evaluation of peritoneal dissemination in individual mice are shown in Figure 17. Although the actual value of the amount of ²¹¹At administered could not be obtained due to malfunction of a calibrator, it was estimated based on calculations of the half-life, etc. that approximately 0.3 and 0.6 MBq were administered to each mouse (hereafter, the doses are referred to as "0.3 and 0.6 MBq"). Compared to the untreated group, the group administered with ²¹¹At alone showed no sufficient effects at either the low dose (0.3 MBq) or the high dose (0.6 MBq). On the other hand, in the hNIS-DPC/²¹¹At administration group, a degeneration effect on peritoneal dissemination was observed at 0.6 MBq (the hNIS-DPC/211At administration group showed a score of 1.4, compared to an average score of 3.2 for the untreated group), although sufficient medicinal effects were not observed at 0.3 MBq. Therefore, it was considered that hNIS-DPCs accumulated in the vicinity of the cancer tissues and incorporated ²¹¹At therein, and as a result, the hNIS-DPCs exhibited cytotoxicity on surrounding cells (i.e., degeneration and death of the cancer cells), which affected the score (i.e., the score was lowered). These results demonstrated that hNIS-DPCs functioned as delivery carriers for ²¹¹At and exhibited sufficient toxicity on the cancer, compared to the case of administration of ²¹¹At alone.

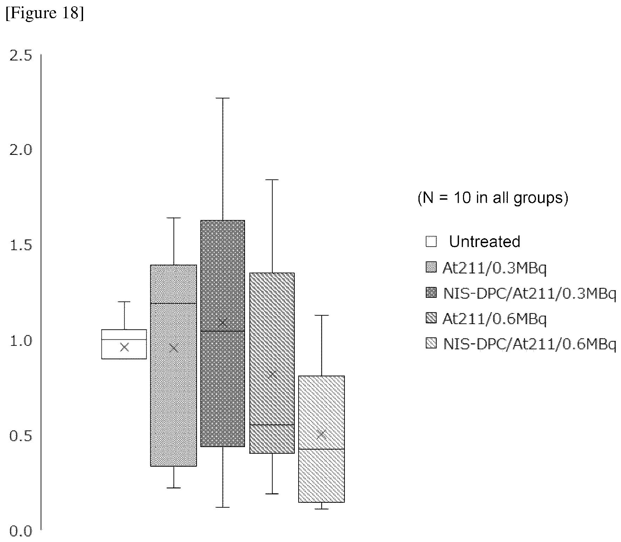

In addition, the results obtained by evaluating the toxicity of hNIS-DPCs and ²¹¹A administration against cancer tissues, using the expression level of FGFR4 gene by qPCR as an indicator, are shown in Figure 18. The results showed a similar tendency to the evaluation results obtained by visual observation. That is to say, while ²¹¹At alone did not show a sufficient cancer degeneration effect, the hNIS-DPC/²¹¹At 0.6MBq administration group showed a sufficient cancer degeneration effect, compared to the untreated group.

From the aforementioned results, it became clear that hNIS-DPCs can be used as carriers to transport ²¹¹At to peritoneal dissemination. Furthermore, it became clear that even at a dose where ²¹¹At alone does not show any cancer regression effect, when it is administered together with hNIS-DPCs, it efficiently accumulates in the vicinity of the peritoneal dissemination and exerts a cancer regression effect, that is, that hNIS-DPCs enhances the cancer regression effect of ²¹¹At.

### 6. Evaluation of the influence of ²¹¹At on cancer tissues when ²¹¹At is used as a radionuclide (2)

Next, whether the enhancement of the antitumor effects of hNIS-DPCs is observed dependently by the dose (the number of administrations) of the hNIS-DPCs was confirmed. In addition to the three times of administrations performed in 5 above, one and two times of administrations were set regarding the number of administrations, and a comparison was then made in terms of the influence of the number of administrations on cancer tissues. It is to be noted that, regarding the number of cells administered, since a significant difference was observed from the untreated group even when the number of cells was set to be 0.5 x 10⁶ cells/body (data not shown), 0.5 x 10⁶ cells/body, which was half the number of cells administered in 5 above (1 x 10⁶ cells/body), was administered from the present experiment onwards. The amount of ²¹¹At administered was set to be 0.6MBq/body.

### 6-1. Methods

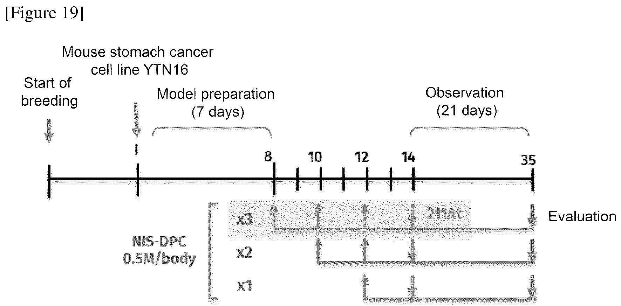

As group configuration, with regard to the peritoneal dissemination models, the following 5 groups were established: (1) an untreated group (n = 10), (2) a ²¹¹At (0.6 MBq/body) administration group (n = 10), (3) an hNIS-DPCs (one time) + ²¹¹At (0.6 MBq/body) administration group (n = 10), (4) an hNIS-DPCs (2 times) + ²¹¹At (0.6 MBq/body) administration group (n = 10), (5) hNIS-DPCs (3 times) + ²¹¹At (0.6 MBq/body) administration group (n = 10). The timing of cell administration is shown in Figure 19. The cells administered were prepared by thawing frozen cells, and the number of cells administered was set to be 0.5 x 10⁶ cells/body. After the cells had been administered one to three times, ²¹¹At was administered once. Regarding the route of administration, both the cells and ²¹¹At were intraperitoneally administered. The day of ²¹¹At administration was set to be Day 0, and the mice were euthanized 21 days later. The amount of tumor particles present in the abdominal cavity was evaluated and scored. The scores were set to be 5 levels (4: large tumor particles present over the entire surface, 3: some tumor particles present but not over the entire surface, 2: small tumor particles clearly present, 1: only a few tumor particles can be confirmed by close observation, 0: no tumor particles present). A score of 4 was given if a large amount of peritoneal dissemination remained, and a score of 0 was given if such peritoneal dissemination had almost disappeared. Box plots were produced based on the scores of individual groups, and the presence or absence of significant differences among the three groups was evaluated using a Tukey test.

### 6.2. Results

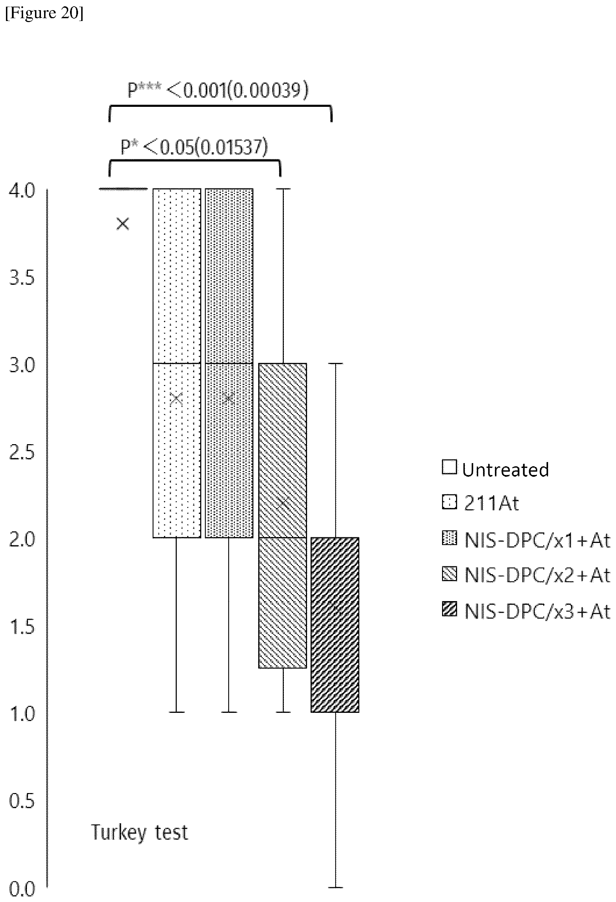

After administration of ²¹¹At, individual mice were observed for 21 days, and the evaluation results of the scoring by visual observation are shown in Figure 20. When compared with the untreated group, no significant difference was observed in the group administered with ²¹¹At alone (²¹¹At) and the group administered with the cells once (NIS-DPC/x1+At), but a difference was observed in the group administered twice (NIS-DPC/x2+At) and the group administered three times (NIS-DPC/x3+At), and it was recognized that the antitumor effects were enhanced depending on the number of administrations.

These results demonstrate that the antitumor effects depend on the number of administrations of NIS-DPCs (the number of administered cells), and it was confirmed that NIS-DPCs are essential for the antitumor effects to be exhibited. It was also suggested that a certain amount or more of NIS-DPCs needs to accumulate in the vicinity of the tumor tissue in order for the antitumor effects to be exhibited.

### 7. Evaluation of medicinal effects of hNIS-DPCs using ²¹¹At based on survival curves

### 7.1. Methods

As group configuration, with regard to the peritoneal dissemination models, the following three groups were established: (1) an untreated group (n = 16), (2) a ²¹¹At (0.6 MBq/body) administration group (n = 17), and (3) an hNIS-DPC/²¹¹At (0.6 MBq/body) administration group (n = 17). In addition, the following two groups were established for normal mice: (1) a ²¹¹At administration group (n = 3) and (2) an hNIS-DPC/²¹¹At administration group, and the influence of ²¹¹At alone and ²¹¹At/hNIS-DPCs on the survival rate of the mice were also evaluated.

The cells administered were prepared by thawing frozen cells, and the number of cells administered was set to be 0.5 x 10⁶ cells/body. After the cells had been administered three times, ²¹¹At was administered once. Regarding the route of administration, both the cells and ²¹¹At were intraperitoneally administered. The day of ²¹¹At administration was set to be Day 0, and the individuals in each group were observed, and the presence or absence of death thereof was confirmed. The observation period was 49 days. Kaplan-Meier curves were produced from the survival hours (days) of individual mice, and the presence or absence of significant differences among the groups was evaluated using a Log-rank test. When individual mice died and after the mice were euthanized at the time of completion of the observation period, the amount of tumor particles present in the abdominal cavity was evaluated and scored. The scores were set to be 5 levels (4: large tumor particles present over the entire surface, 3: some tumor particles present but not over the entire surface, 2: small tumor particles clearly present, 1: only a few tumor particles can be confirmed by close observation, 0: no tumor particles present). A score of 4 was given if a large amount of peritoneal dissemination remained, and a score of 0 was given if such peritoneal dissemination had almost disappeared. Box plots were produced based on the scores of individual groups, and the presence or absence of significant differences among the three groups was evaluated using a Tukey test.

### 7.2. Results

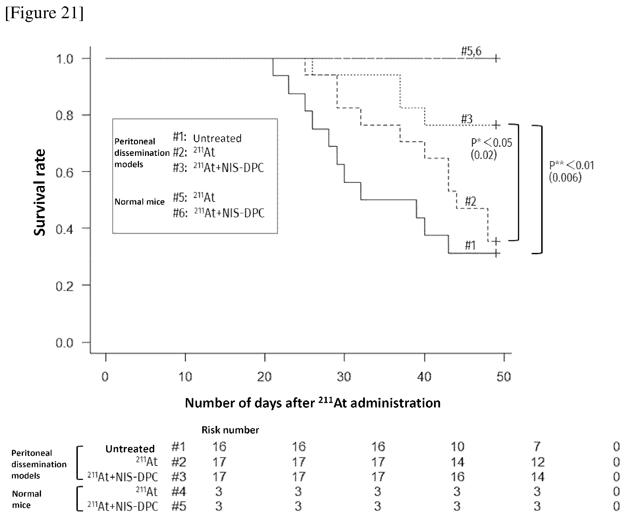

After administration of ²¹¹At, individual mice were observed for 49 days, and the survival period was evaluated and analyzed using a Kaplan-Meier curve and a Log-rank test. The results are shown in Figure 21. In the untreated group (#1: Untreated), dead mice were observed from the 21st day, and the median value indicating a 50% survival rate was 35.5 days (solid line). In the group administered with ²¹¹At alone (#2: ²¹¹At), dead mice were observed from the 25th day. The number of dead mice increased more slowly than in the untreated group, with the median value being 44 days (large dotted line), but at the end of the observation period, there was no significant difference in survival rate from the untreated group. On the other hand, the survival rate was extended in the ²¹¹At and NIS-DPCs administration group (#3: ²¹¹At+NIS-DPC) compared with the untreated group and the group administered with ²¹¹At alone, a significant difference was observed to the two above groups (vs. untreated group: P = 0.00583; vs. ²¹¹At: P = 0.02). In addition, in normal mice administered with ²¹¹At alone or with ²¹¹At/hNIS-DPCs, toxicity or findings that would affect the survival rate were not observed in both cases, and all individuals could be confirmed to survive during the observation period.

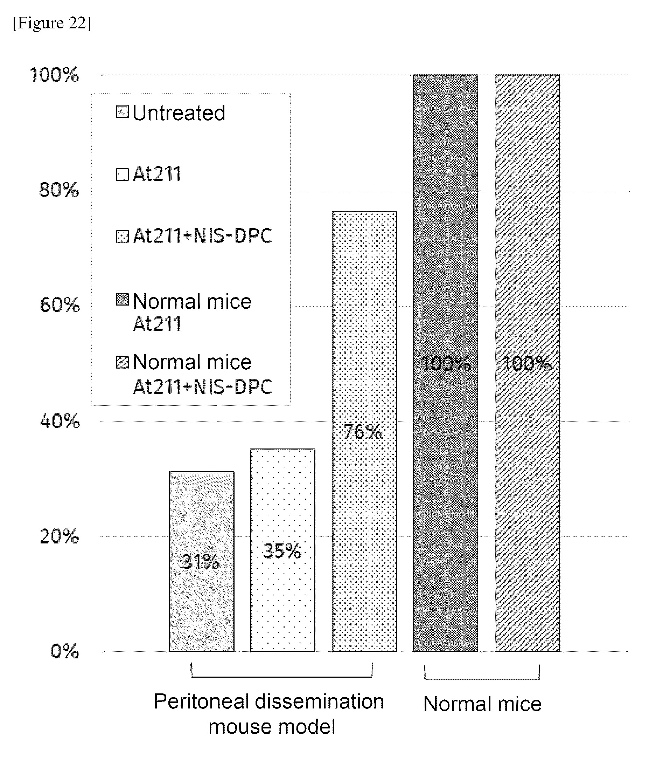

The survival rates after the end of the observation period in each of the above-described groups are summarized in Figure 22. The ²¹¹At+NIS-DPCs administration group showed a higher survival rate than the untreated group and the ²¹¹At alone administration group.

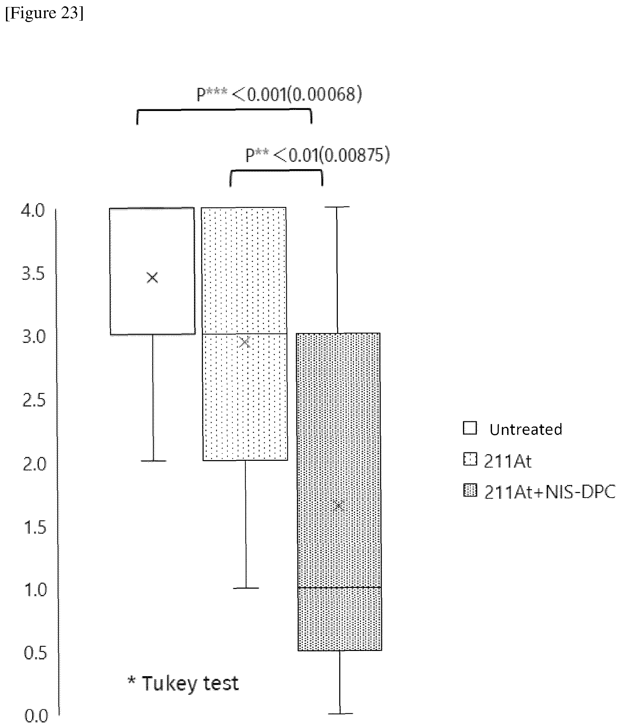

The evaluation results of peritoneal dissemination in the above-described peritoneal dissemination models by scoring by visual observation are shown in Figure 23. When compared with the untreated group, no significant difference was observed in the ²¹¹At alone administration group (²¹¹At), but a significant difference was observed in the ²¹¹At and NIS-DPCs administration group. Similarly, when compared with ²¹¹At as well, a significant difference was observed, although not as strong as when compared with the untreated group.

From the above-described results, it was considered that the administered hNIS-DPCs accumulated in the vicinity of the cancer tissues and incorporated ²¹¹At therein, which caused toxicity to the surrounding cells (i.e., the regression and death of cancer cells), thereby affecting the score (i.e., the score was reduced). It was also suggested that the survival rate was extended due to the regression and death of cancer cells. From these results, it was confirmed that hNIS-DPCs functioned as delivery carriers of ²¹¹A and exhibited sufficient toxicity to cancer, which was considered to contribute to extension of the survival rate.

### 8. Generalization

It was suggested from the results of qPCR and immunostaining that DPCs migrate and accumulate in tissues around cancer cells, regardless of the route of administration, such as intraperitoneal or intravenous administration, etc. Similarly, it could also be confirmed that DPCs, into which hNIS has been introduce by lentivirus and whose function has been modified (hNIS-DPCs), also migrate and accumulate in the vicinity of cancer cells, and that the properties of DPCs are maintained. In addition, since incorporation of the radioisotopes ¹²⁵I and ²¹¹At into hNIS-DPCs was confirmed, it was assumed that the iodine transporter introduced into DPCs maintains its function. Moreover, since degeneration of cancer cells by the combination of hNIS-DPCs and ²¹¹At was observed as a result of a toxicity evaluation test of applying ²¹¹At , which emits α rays, to the cancer cells, the PoC (Proof of Concept) of internal radiation therapy using hNIS-DPCs and α rays could be confirmed. The present treatment method is expected to be a novel treatment option for refractory cancers, such as peritoneal dissemination.

### Industrial Applicability

The therapeutic composition according to the present invention exhibits effects for the treatment of cancer, in particular, refractory cancer (e.g., peritoneal dissemination, etc.). Therefore, the present invention is expected to be utilized in the medical field (in particular, in the field of cancer treatment).

## Claims

1. Dental pulp stem cells, which express a sodium iodide symporter (NIS).

2. The cells according to claim 1, which are derived from a human dental pulp.

3. The cells according to claim 1, wherein the NIS is introduced from the outside.

4. A composition comprising the cells according to any one of claim 1 to claim 3, wherein the composition is **characterized in that** it is used in combination with a radionuclide.

5. The composition according to claim 4, which is a pharmaceutical composition for use in the treatment of a cancer.

6. The composition according to claim 5, which is **characterized in that** the composition is intratumorally, intraperitoneally or intravenously administered, and the radionuclide is intratumorally, intraperitoneally, intravenously or orally administered.

7. The composition according to claim 5, wherein the radionuclide is any of ¹³¹I, ²¹¹At or ¹⁸⁸Re.

8. The composition according to claim 5, wherein the cancer is a peritoneal dissemination.

9. The composition according to claim 4, which is a composition for use in the diagnosis of a cancer.

10. The composition according to claim 9, which is **characterized in that** the composition is intratumorally, intraperitoneally or intravenously administered, and the radionuclide is intratumorally, intraperitoneally, intravenously or orally administered.

11. The composition according to claim 9, wherein the radionuclide is any of ¹³¹I, ¹²³I, ¹²⁵I, ¹²⁴I or ^{99m}Tc.

12. The composition according to claim 9, wherein the cancer is a peritoneal dissemination.
